Europäisches Patentamt

European Patent Office

Office européen des brevets

(19)

(11) Publication number: 0 233 389

A1

(12) **EUROPEAN PATENT APPLICATION**

(21) Application number: 86300972.6

(51) Int. Cl.⁴: G01N 23/16

(22) Date of filing: 12.02.86

(43) Date of publication of application:
26.08.87 Bulletin 87/35

(84) Designated Contracting States:
CH DE GB LI SE

(71) Applicant: Repsch, Josef W.
10325-61 Avenue
Edmonton Alberta T6H 1K9(CA)
Applicant: Repsch, Jan M.
10325-61 Avenue
Edmonton Alberta T6H 1K9(CA)
Applicant: Liska, Frank F.
10325-61 Avenue
Edmonton Alberta T6H 1K9(CA)

(72) Inventor: Repsch, Josef W.
10325-61 Avenue
Edmonton Alberta T6H 1K9(CA)
Inventor: Repsch, Jan M.
10325-61 Avenue
Edmonton Alberta T6H 1K9(CA)
Inventor: Liska, Frank F.
10325-61 Avenue .
Edmonton Alberta T6H 1K9(CA)

(74) Representative: Haggart, John Pawson et al
Page, White & Farrer 5 Plough Place New
Fetter Lane
London EC4A 1HY(GB)

(54) **A method of measuring the weight per unit area, density and thickness of a moving sheet.**

(57) Method and apparatus for measuring the weight per unit area and providing cross-sectional, longitudinal, and diagonal profiles of density or thickness of moving manufactured sheet products. The apparatus comprises a stationary radiation source (2), an array of detectors (4), and electronic means (9, 10, 11) for processing the signals from the detectors (4) to obtain a total cross-sectional, longitudinal, and diagonal profile of density or thickness of moving manufactured sheet products. The energy output of the radiation source (2) remains constant during a measuring operation. The apparatus utilizes a line-up of individual radiation sources delivering constant radiation, and each individual radiation source generates an equal amount of radiation. The radiation transmitted from the individual radiation source is intercepted by the detectors (4) after penetrating the sheet product (3). The electronic means includes an interface unit, signal processor and numerical display units.

FIG. 1

# A method of measuring the weight per unit area, density and thickness of a moving sheet

This invention relates to method and apparatus for performing measurements of weight per unit area, and providing cross-sectional, longitudinal, and diagonal profiles of density or thickness of moving manufactured sheet products. In particular, it relates to such measurements where the sheet products are, for example agglomerated boards made of wood chips, flakes, strands, or wood fiber; ligno-cellulosic materials; and other organic and inorganic substances.

At the present time such measurements are performed by employing scanning devices comprising a radiation source located beneath the longitudinaly moving products, and an appropriate sensing device mounted above and in precise vertical alignment with the radiation source. The movement of the scanner is such that the radiation source and the sensing device create a zig-zag measuring pattern over the longitudinally moving products.

The measurements performed by the method utilizing the zig-zag pattern provide only single spot readings of the measured variable which depicts the density or thickness at the given cross-section of the advancing products. It is thus impossible to obtain a full profile of the measured variable at a given cross-section. The method also does not provide total readings of the longitudinal profile of the measured variable of the manufactured products.

Other measuring methods well known in this art provide only averaging of the spot readings of the measured variables within the manufactured sheet products. For this reason, these methods do not provide adequate data for reconstructing the total cross-sectional and longitudinal profiles of the measured variables.

The above methods also do not provide means for identifying the exact coordinates of the measured variables at any given point within the manufactured sheet products. They also require frequent recalibration of the measuring equipment because of varying process conditions and deterioration of the measuring devices.

This invention seeks to provide a method and an apparatus for more effective measurements of thickness and density of manufactured sheet products.

Accordingly the invention is a method of measuring the weight per unit area, density and thickness of a moving sheet by applying the techniques of penetrating radiometry the method comprising passing the sheet between a stationary linear array of detectors to take a plurality of simultaneous spot readings transverse to the direction of motion of the sheet, said stationary linear array of detectors taking readings in regular increments of time as the sheet advances longitudinally, said regular increments of time being synchronized by a sequence of pulses generated by a linear encoder and being determined by the resolution of said linear encoder, said simultaneous transverse spot readings being taken consecutively as the sheet advances to give a grid-like pattern of spot measurements covering the entire area of the advancing sheet and converting said spot measurements to electronic signals, processing those signals by a data acquisition system, interfaced with a computer adapted to generate data for the physical presentation of the results.

In a further aspect the invention is an apparatus to measure the weight per unit area, density and thickness of a moving sheet, the apparatus comprising a radiation source; a collimator for the radiation from said source; a linear array of radiation detectors spaced from said source, all said detectors being at the same distance from the source and spaced from each other; means for aligning said source, collimator and detectors in a plane; means to transmit a signal from the detectors; means to receive the signal; means to process the signal; and means to display the processed signal.

It is an object of this invention to provide a method of identifying the entire transverse profiles of the measured variable in relation to the encoded position identified by the horizontal coordinates of the consecutive detector and defined number of pulses generated by the linear encoder.

It is a further object of this invention to provide a method for identifying the entire longitudinal profile of measured variable in relation to the encoded position determined by only one of the detectors and defined number of pulses generated by the linear encoder.

It is also an object of this invention to provide a method for identifying any combination of the diagonal profiles of measured variable in relation to any encoded position determined by the horizontal coordinates of the detectors and defined number of pulses generated by the linear encoder.

It is still another object of this invention to provide a method for identifying the average value of the measured variable within the manufactured sheet products.

It is another object of this invention to provide software and hardware means allowing for display and presentation of the acquired data.

Further objects of our invention will appear from a detailed description of an embodiment. It is to be understood, however, that the present invention is in no way limited to the details of such embodiment, but is capable of numerous modifications within the scope of the appended claims.

Figure 1 is a block diagram of the apparatus embodying the invention.

Figure 2 is a diagrammatic side view of the longitudinally advancing products and the stationary measuring assembly.

Figure 3 shows the concept of identifying the coordinates of the measured variables.

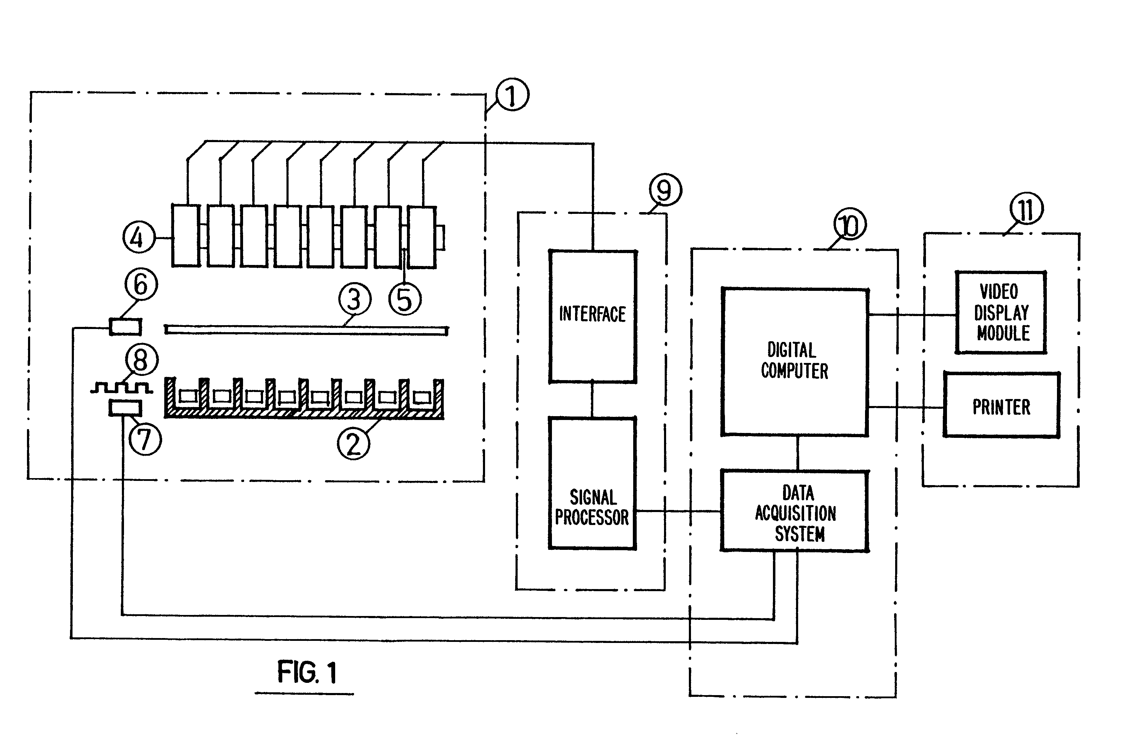

A preferred embodiment of the novel apparatus for measuring of weight per unit of manufactured sheet products is shown diagrammatically in Figure 1.

All mechanical, electrical, and electronic components of the apparatus are well known in the industry and need not be described in detail.

The detector/electronic assembly, interface and power supply module, and signal processor and display unit, depicted on Figure 1 as items 4 and 7 are disclosed in the patent application filed by R.A. Tawil, A. Scalanczy, K. Velbeck, J. Chamberlain, D. Leslie, and C.W. King; all of Harshaw/Filtrol Partnership, of Solon, Ohio.

At the time of filing this patent, any reference numbers related to the patent application of Harshaw/Filtrol were not available. It is known to us that the patent of Harshaw/Filtrol was filed with the United States Patent Office.

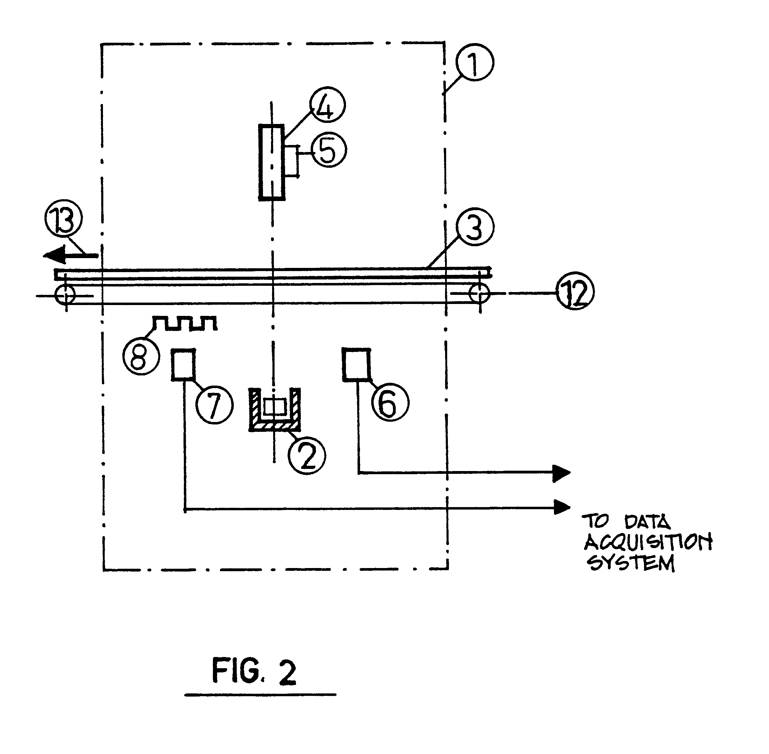

The sheet products 3 are carried over the array of detectors by transporting devices comprising sets of rolls, endless belts, or other conveying means 12 in Figure 2. The conveying means for transporting sheet products are mounted on a supporting frame which is not shown and is not a part of this invention.

All components of the measuring assembly are disposed on the frame 1, shown symbolically.

The radiation source 2, located beneath the advancing sheet product, comprises a line of individual compartments each containing a radiation substance. Each individual compartment is in precise vertical and horizontal alignment with the detector/electronic assembly.

The radiation substance used in this invention may be any type of radiation source emitting Gamma or Beta rays, X-rays or neutrons.

The individual detectors 4 are disposed on the base 5 in such a way that their displacement allows for reception of the full amount of the emitted radiation from the radiation source 2 and conversion of the received radiation into readable electrical signals as it is shown in Figure 1.

The cross-sections of the radiation source 2 and detectors 4 are disposed within the same plane.

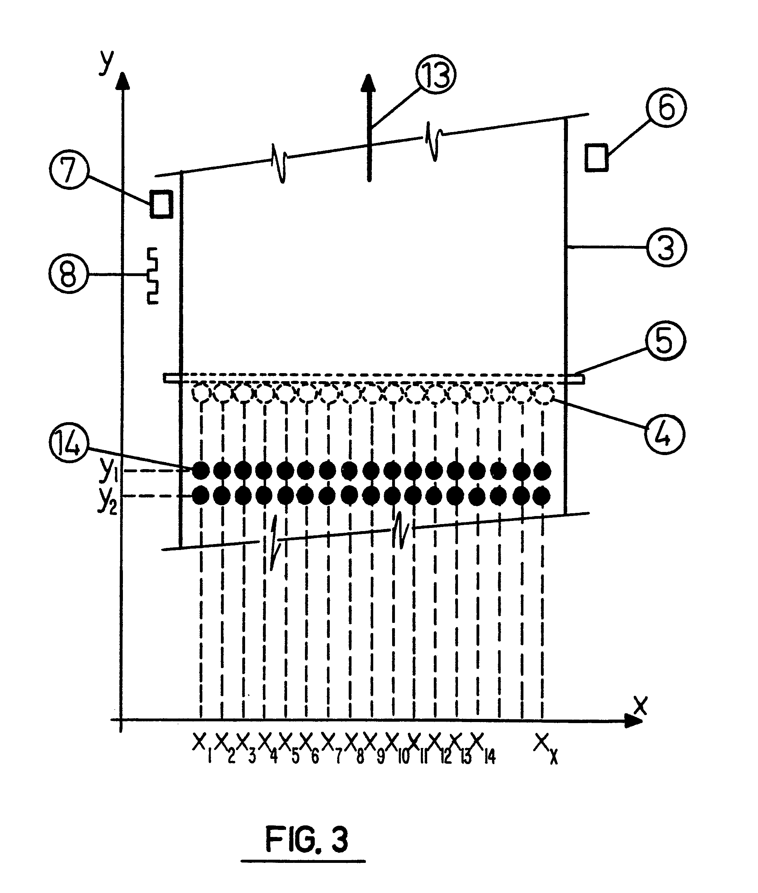

The detectors 4 provide discrete coding of the coordinates $x_i$ to $x_n$ across the manufactured sheet products as shown in Figure 3.

A linear encoder 7, a device well known in the industry, is disposed on the frame 1 of the measuring assembly to provide a sequence of pulses 8 encoding the longitudinal movement 13 of the manufactured sheet products as shown in Figure 3.

Transverse, discrete encoding of the advancing sheet products 3 by the detectors 4, together with the longitudinal encoding of the movement of the sheet products by the sequence of the pulses 8 create a grid-like pattern of spot measurements 14 over the advancing sheet products 3 as shown in Figure 3.

The base 5 must be of such a shape that allows for the installation of detectors 4 at an equal distance from the radiation source 2.

The proximity switch 6 is a device well known in the industry and is disposed on the frame 1 of the measuring assembly. The proximity switch 6 generates an electrical impulse when it detects the presence of the advancing sheet products.

The assemblies 9, 10, and 11 as they are shown in Figure 1, comprise electronic components well known in the industry for the acquisition, processing and display of electric and electronic signals received from the detectors 4.

The linear encoder 7, proximity switch 6, and detectors 4 are connected by appropriate means allowing for the transmission of electrical and electronic signals over the distance between the above devices and the data acquisition system. The data acquisition system is an integral part of the electronic data processing assembly as shown in Figure 1.

It is well known that the measurements of the weight per unit area can be performed by penetrating radiometry techniques based on the following equation:

$$I = I_o e^{-\mu x} \quad (1)$$

where I is the transmitted intensity of the radiation, $I_o$ is the intensity of the incident radiation, e is the natural log base, $\mu$ equals the linear attenuation coefficient, and x is the weight per unit area of the measured material. The equation (1) may be presented in the following form:

$$x = \frac{1}{\mu} \ln \frac{I_o}{I} \quad (2)$$

The weight per unit area x as described by the equation (2) may be defined also as a product of the density, expressed as weight per unit volume, and thickness, expressed as units of length, as:

$$x = d \bullet t \quad (3)$$

where x is the weight per unit area, d is the density of the product, and t is the thickness of the product.

The apparatus is calibrated in terms of "weight per unit area" and if the product density is maintained constant, the results of the measurements are presented as thickness of the measured product.

Conversely, if the thickness of the product is maintained constant, the results of the measurements are expressed as the density of the measured product.

The calibration techniques to be applied for calibrating the apparatus are well known in the industry and need not be described.

From the above it will be readily understood that the apparatus described will attain the objects of the invention.

**Claims**

1. A method of measuring the weight per unit area, density and thickness of a moving sheet by applying the techniques of penetrating radiometry, comprising passing a sheet (3) relative to a stationary linear array of detectors (4) to take a plurality of simultaneous spot readings transverse to the direction of motion (13) of the sheet, the stationary linear array of detectors (4) taking readings in regular increments of time as the sheet advances longitudinally, which regular increments of time are synchronized by a sequence of pulses (8) generated by a linear encoder (7) and being determined by the resolution of said linear encoder, the simultaneous transverse spot readings being taken consecutively as the sheet (3) advances to give a grid-like pattern (14) of spot measurements covering the entire area of the advancing sheet, and converting these spot measurements to electronic signals, processing those signals by a data acquisition system, interfaced with a computer adapted to generate data for the physical presentation of the results.

2. A method as claimed in Claim 1, in which the variation or the average of density or thickness of each longitudinally encoded transversal profile of spot measurements is presented.

3. A method as claimed in Claim 1, in which the variation or the average of density or thickness of a preselected group of longitudinally encoded transverse profiles of spot measurements is presented.

4. A method as claimed in Claim 1, in which the variation or the average of density or thickness of each transversally encoded longitudinal profile of spot measurements is presented.

5. A method as claimed in Claim 1, in which the variation or the average of density or thickness of a preselected group of transversely encoded longitudinal profiles of spot measurements is presented.

6. A method as claimed in Claim 1, in which the computer is adapted to generate data for presentation as selected measured values of density or thickness in the form of a contour map or as the percentage of the total area of the manufactured sheet products; or as the total average of density or thickness of the manufactured sheet products; or as a display of progressive deterioration and the rate of progressive deterioration of a measured value of the sheet.

7. Apparatus to measure the weight per unit area, density and thickness of a moving sheet, the apparatus comprising a radiation source (2) including a collimator for the radiation from that source, a linear array of radiation detectors (4) spaced from the source (2), all the detectors (4) being at the same distance from the source and spaced from each other, means for aligning the source and collimator (2) and detectors (4) in a plane, means - (9) to transmit a signal from the detectors (4), means (10) to receive and process the signal, and means (11) to display the processed signal.

8. Apparatus as claimed in Claim 7, in which the radiation source is a source of nuclear radiation.

9. Apparatus as claimed in Claim 7, in which the nuclear source is a source of X-rays, gamma-rays or beta-rays.

10. Apparatus as claimed in Claim 7, in which the collimator collimates the emitted radiation to a strip of preset thickness, and the detectors (4) are disposed to receive the full amount of the emitted radiation.

11. Apparatus as claimed in any one of Claims 7 to 10, in which one detector is taking continuous readings of the incident radiation for the purpose of calibration.

12. Apparatus as claimed in any one of Claims 7 to 11, including a linear encoder (7) operable to generate a sequence of pulses (8) encoding longitudinal movement of the sheet.

13. Apparatus as claimed in any one of Claim 7 to 12, in which a proximity switch (6) senses the presence of a passing sheet and is operable to trigger the radiation source (2).

14. Apparatus as claimed in any one of Claims 7 to 13, in which the means (10) to process the signal is a computer, and the means (11) to display the processed signal is a video screen or a printer.

FIG. 1

FIG. 2

TO DATA
ACQUISITION
SYSTEM

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 618 906  (HEATH LTD.)<br><br>* Pages 4-6,10,13,18; figures 1,2<br>* & GB-A-1 552 224<br><br>--- | 1,7,9-11 | G 01 N  23/16 |
| X | US-A-3 016 460  (G. ANDRESEN)<br>* Columns 3,6; figure 2 *<br><br>--- | 1,7 | |
| X | US-A-3 489 901  (L. BROWN)<br>* Columns 2,5,6 *<br><br>--- | 1,7 | |
| X | GB-A-2 110 367  (TOKYO SHIBAURA DENKI)<br>* Page 3, right-hand column; page 4 *<br><br>--- | 1,7,11,14 | |
| A | GB-A-1 180 831  (IND. NUCLEONICS CORP.)<br>* Page 1, right-hand column; page 3, left-hand column *<br><br>--- | 2,3 | **TECHNICAL FIELDS SEARCHED (Int. Cl.4)**<br><br>G 01 N  23/16<br>G 01 B  15/02<br>G 01 G   9/00<br>G 01 N   9/24 |
| A | GB-A-1 254 432  (IND. NUCLEONICS CORP.)<br>* Page 3; page 4, left-hand column; page 7, right-hand column; page 13, claims 1-6 *<br><br>--- | 2,3,6 | |
| A | US-A-4 223 346  (G. NEIHEISEL et al.)<br>* Columns 3,6; figure 7 *<br><br>----- | 1,12 | |

The present search report has been drawn up for all claims

| Place of search | Date of completion of the search | Examiner |
|---|---|---|
| THE HAGUE | 14-10-1986 | BOEHM CH.E.D. |